# FASCICULE DE BREVET EUROPEEN

(11) **EP 3 063 123 B1**
(45) Date de publication et mention de la délivrance du brevet: **30.05.2018**
(21) Numéro de dépôt: 14814915.6
(22) Date de dépôt: 21.10.2014
(51) Int. Cl.: C07C 67/08, C07C 67/54, C07C 67/58, C07C 69/54, C07C 45/52, C07C 47/22, C07C 51/44, C07C 57/04, C07C 51/487

(54) **PROCEDE DE PRODUCTION D'ESTERS (METH)ACRYLIQUES LEGERS**
VERFAHREN ZUR HERSTELLUNG LEICHTER (METH)ACRYLSÄUREESTER
PROCESS FOR PRODUCING LIGHT (METH)ACRYLIC ESTERS

(30) Priorité: 29.10.2013 FR 1360541; 10.02.2014 FR 1450994
(43) Date de publication de la demande: 07.09.2016
(73) Titulaire: Arkema France, 92700 Colombes (FR)
(72) Inventeur: TRETJAK, Serge, F-57520 Roulhing (FR); DENIS, Stephane, F-57660 Leyviller (FR); DELAIS, Lionel, 57530 Courcelles Chaussy (FR); MORELIERE, Anne, F-57740 Longeville-les-St-Avold (FR)
(74) Mandataire: Bonnel, Claudine
(86) Numéro de dépôt international: PCT/FR2014/052667
(87) Numéro de publication internationale: WO 2015/063388

(56) Documents cités:
- GB-A- 2 016 461
- US-A- 3 868 410
- US-A- 6 025 520
- US-A1- 2005 107 629

## Description

### DOMAINE TECHNIQUE

La présente invention concerne un perfectionnement à la production en continu de (méth)acrylate de méthyle ou de (méth)acrylate d'éthyle par estérification directe de l'acide (méth)acrylique par du méthanol ou de l'éthanol, dans des conditions de réaction pour lesquelles l'acide est en excès par rapport à l'alcool.

### ART ANTERIEUR ET PROBLEME TECHNIQUE

Il est connu de produire des esters (méth)acryliques, notamment l'acrylate ou le méthacrylate de méthyle, et l'acrylate ou le méthacrylate d'éthyle, par estérification directe de l'acide (méth)acrylique par l'alcool correspondant, catalysée par exemple par l'acide sulfurique ou une résine échangeuse d'ions. On peut citer par exemple les procédés décrits dans les documents US 3,776,947 ; US 3,914,290 ; US 4,664,229 ou US 4,435,594 pour lesquels l'alcool est utilisé en excès par rapport à l'acide (méth)acrylique.

La réaction d'estérification génère de l'eau et s'accompagne généralement de réactions secondaires produisant des impuretés, en particulier des composés lourds, c'est-à-dire présentant une température d'ébullition élevée, supérieure à celle de l'ester recherché.

Dans de tels procédés, pour des raisons environnementales et économiques, il est indispensable de recycler à la réaction les réactifs non réagis - l'alcool majoritairement et l'acide - mais aussi de valoriser les produits lourds générés au cours du procédé, tout en recherchant un produit fini de haute pureté.

A ces fins, on procède généralement à un ensemble de traitement du mélange réactionnel à l'aide de distillations et/ou d'extractions, décantations, ensemble qui est à la fois relativement complexe à mettre en oeuvre, et coûteux sur le plan énergétique, et qui présente l'inconvénient de générer un résidu final non valorisable pouvant représenter une perte en matières premières.

Les problèmes qui se posent lors de la fabrication des esters (méth)acryliques légers, notamment la formation de sous-produits lourds, vont maintenant être exposés, par commodité, sur la base de l'exemple de l'acrylate d'éthyle obtenu par estérification de l'acide acrylique par l'éthanol. Cependant, les problèmes et la solution proposée par l'invention sont les mêmes dans le cas de la mise en oeuvre dans la réaction d'estérification, d'une part, de l'acide méthacrylique, ou d'autre part, de méthanol.

Au titre des réactions secondaires conduisant à la formation de sous-produits lourds au cours de la fabrication de l'acrylate d'éthyle, il s'agit essentiellement de la formation d'oligomères de l'acide acrylique n'ayant pas réagi - acide acrylique dimère (acide 3-acryloxypropionique, n=1) et dans une moindre mesure, acide acrylique trimère (acide 3-acryloxy, 3-propioxy propionique, n=2) -, mais aussi de réactions d'addition de Michael (adduits de Michael), en particulier entre l'acrylate d'éthyle déjà formé et l'éthanol n'ayant pas réagi conduisant à l'éthoxy propionate d'éthyle.

L'éthoxy propionate d'ethyle (EPE) est un sous-produit dit lourd car sa température d'ébullition (168°C, P atmosphérique) est nettement supérieure à celle de l'acrylate d'éthyle (100°C, P atmosphérique), et il se concentre dans le procédé au fur et à mesure de l'avancement de la réaction en même temps que les oligomères de l'acide acrylique. Sa température d'ébullition étant proche de celle de l'acide acrylique (144°C, P atmosphérique), l'EPE va de plus se concentrer dans la boucle de recyclage de l'acide acrylique n'ayant pas réagi, et nécessiter une purge de cette boucle de recyclage ; une part significative d'acide acrylique est alors perdue lors de la purge à moins d'effectuer un craquage thermique du flux purgé pour régénérer l'acide acrylique présent sous forme d'oligomères, ce craquage thermique n'ayant cependant pas d'effet sur l'EPE. Par ailleurs, l'EPE étant le plus léger des sous-produits lourds, sa présence dans le milieu réactionnel va interférer dans la purification finale de l'acrylate d'éthyle.

Il a été proposé dans le procédé de synthèse d'acrylate d'éthyle décrit dans le document US 2005/0107629, d'effectuer une purge du réacteur, le flux purgé étant envoyé sur une unité de distillation séparant un distillat contenant de l'acide acrylique, de l'acrylate d'éthyle et de l'éthanol, qui est recyclé à la réaction, et un résidu qui est soumis à une unité de récupération du catalyseur d'estérification. Ce procédé permet d'éviter une accumulation en sous-produits lourds dans le réacteur, et de recycler les composés directement valorisables présents dans le flux purgé, mais représente une perte significative d'acide acrylique présent sous la forme d'oligomères et/ou d'éthoxy propionate d'éthyle dans le flux purgé.

Dans le document US 6,025,520, il est proposé d'effectuer la réaction d'estérification entre l'acide (méth)acrylique et un alcool comportant de 1 à 3 atomes de carbone, catalysée par une résine échangeuse d'ions fortement acide, sous pression réduite et avec un rapport molaire alcool/acide inférieur à 1, de préférence allant de 0,3 à inférieur à 1. Ces conditions permettent d'améliorer le rendement et la sélectivité de la réaction d'estérification et de réduire significativement la formation de sous-produits lourds tels que le méthoxy propionate de méthyle (dans le cas où l'alcool est le méthanol) dont la présence est en outre problématique pour le train de purification de l'ester recherché.

Cependant, malgré l'avancée apportée par ce procédé, la mise en oeuvre des conditions réactionnelles présente encore de nombreux inconvénients ; d'une part, ce procédé nécessite de travailler sous pression réduite au niveau de l'ensemble réactionnel, et par conséquent nécessite une technologie particulière de réacteur car le milieu réactionnel devient biphasique (gaz/liquide) ou même triphasique dans des conditions de catalyse hétérogène ; d'autre part, la vitesse volumique horaire (VVH), du flux soumis à la réaction, définie par le rapport entre le débit de flux réactif et le volume de catalyseur va de 0,1 h⁻¹ à moins de 1 h⁻¹, ce qui signifie qu'à production d'ester égale, la quantité de catalyseur mise en oeuvre peut varier d'un facteur 10 ; ou ce qui correspond à un temps de séjour dans le réacteur de plus d'une heure et pouvant aller jusque dix heures. Les exemples illustrant ce procédé, ainsi que l'exemple comparatif effectué sous pression atmosphérique ont été réalisés avec une VVH de 0,33 h⁻¹ correspondant à une durée de séjour de 3 heures dans le réacteur.

Le document GB 2 016 461 décrit une méthode pour produire un ester par réaction d'estérification d'un alcool avec un excès d'acide. La méthode est illustrée avec les acides isobutyrique et méthacrylique. La synthèse du méthacrylate de méthyle est effectuée en particulier avec un flux contenant 11% pds de méthanol et 88,8% pds d'acide méthacrylique, correspondant à un excès stoechiométrique en acide d'environ 3, et avec un temps de séjour de 20 minutes dans le réacteur. Le méthacrylate de méthyle purifié est séparé après un procédé de purification en deux étapes mettant en oeuvre deux colonnes de distillation.

Il subsiste donc un réel besoin de pallier les inconvénients des procédés de synthèse de (méth)acrylates légers de l'art antérieur, notamment de minimiser la formation d'éthoxy propionate d'éthyle dans le cas de la synthèse de l'acrylate d'éthyle, préjudiciable pour le bilan matière du procédé (perte de matières premières) et pour la pureté du produit recherché (complexité du train de purification), dans une configuration de procédé simplifiée.

De façon surprenante, les inventeurs ont découvert qu'il était possible de réduire significativement la formation d'éthoxy propionate d'éthyle dans une technologie de réacteur à lit fixe classique en effectuant la réaction d'estérification dans des conditions où l'acide est en excès par rapport à l'alcool et sous pression atmosphérique. Avec ces conditions, il est possible d'utiliser les réacteurs à lit fixe déjà utilisés pour les réactions effectuées en excès d'alcool, puisque la nature du flux réactif maintenu en phase liquide ainsi que le temps de séjour nécessaire ne sont pas modifiés.

De plus, les inventeurs ont trouvé que la formation d'adduits pouvait encore être réduite de façon significative en minimisant la quantité d'eau introduite dans le réacteur, l'eau pouvant provenir de l'alimentation en alcool ou du recyclage de flux comportant de l'acide non réagi et/ou de l'alcool non réagi avec une fraction de l'eau générée par la réaction d'estérification.

Un des objectifs de la présente invention est donc de fournir un procédé de synthèse d'acrylate d'éthyle, et plus généralement de (méth)acrylate de méthyle ou d'éthyle, minimisant la formation d'éthoxy propionate d'éthyle au cours de la réaction d'estérification, ce procédé conduisant à l'amélioration du bilan matières, une simplification du train de purification de l'ester recherché, et une optimisation du bilan énergétique.

Le procédé de l'invention est particulièrement avantageux par rapport au procédé décrit dans le brevet US 6,025,520 puisque la réaction d'estérification est mise en oeuvre à la pression atmosphérique avec des vitesses volumiques horaires plus élevées conduisant à une productivité supérieure.

### RESUME DE L'INVENTION

La présente invention a pour objet un procédé de synthèse de (méth)acrylate de méthyle ou d'éthyle par estérification de l'acide (méth)acrylique avec l'alcool correspondant, en présence d'un catalyseur acide, caractérisé en ce que la réaction d'estérification est effectuée dans un réacteur avec un rapport molaire acide/alcool compris entre 1,05 et 4 et sous une pression allant de la pression atmosphérique à 5 bars, et avec un temps de séjour du flux soumis à la réaction compris entre 30 minutes et 1 heure.

Par « compris entre », on entend bornes comprises au sens de l'invention.

Par acide (méth)acrylique, on entend l'acide acrylique ou l'acide méthacrylique. De préférence, l'acide réactionnel est l'acide acrylique.

De préférence, l'alcool est l'éthanol.

La température de réaction est généralement comprise entre 60°C et 90°C.

Le procédé selon l'invention est avantageusement mis en oeuvre dans une technologie de réacteur à lit fixe, de préférence avec un catalyseur solide du type résine échangeuse d'ions, ou un réacteur agité de préférence avec un catalyseur liquide du type acide sulfurique ou acide organique sulfonique.

La vitesse volumique horaire pour le flux réactionnel est comprise entre 1 h⁻¹ et 2 h⁻¹.

Selon un mode de réalisation, le procédé selon l'invention peut comporter des étapes de déshydratation de flux, par toute technique connue, permettant de réduire la teneur en eau à l'entrée du réacteur ce qui a pour effet de réduire la formation d'adduits.

Selon ce mode de réalisation, le procédé comprend au moins une étape de déshydratation d'un flux alimentant le réacteur.

Ladite étape de déshydratation peut être appliquée à l'un au moins des flux suivants : le flux d'alimentation en alcool frais, un flux de recyclage de l'alcool non réagi, ou un flux de recyclage de l'acide non réagi.

Selon un mode de réalisation, le procédé selon l'invention comprend les étapes suivantes :
a) l'alimentation d'un réacteur en acide (méth)acrylique, en catalyseur acide et en alcool et la réaction d'estérification dans le réacteur telle que définie précédemment ;
b) le soutirage d'un flux de (méth)acrylate de méthyle ou d'éthyle en sortie du réacteur ;
c) la distillation du flux de (méth)acrylate de méthyle ou d'éthyle, permettant de séparer, en tête, un flux de (méth)acrylate appauvri en sous-produits lourds et en acide (méth)acrylique non réagi, et en pied un flux comportant les sous-produits lourds, l'acide (méth)acrylique non réagi et des traces d'alcool et de produits légers ;
d) la séparation du flux de pied obtenu à l'étape c), en un flux comportant l'acide (méth)acrylique non réagi et des traces d'alcool et de produits légers, ce flux étant recyclé dans le réacteur, et en un flux de sous-produits lourds qui est soumis à un craquage thermique libérant un flux de produits valorisables qui retourne à l'étape d) ;
e) l'extraction liquide / liquide du flux de tête obtenu à l'étape c) par un flux aqueux permettant de séparer une phase organique purifiée de (méth)acrylate de méthyle ou d'éthyle, et une phase aqueuse, la phase aqueuse étant distillée pour récupérer d'une part une fraction riche en alcool qui est recyclée vers le réacteur, et d'autre part une fraction riche en eau qui est utilisée en tant que flux aqueux dans l'étape d'extraction liquide / liquide.

Le procédé selon l'invention peut comprendre en outre une ou plusieurs étapes de distillation de la phase organique purifiée de (méth)acrylate de méthyle ou d'éthyle afin d'éliminer des composés organiques supplémentaires et atteindre la pureté recherchée.

Le procédé selon l'invention peut comprendre en outre une étape f) comprenant la déshydratation de l'un au moins des flux suivants : l'alimentation en alcool de l'étape a), le flux comportant l'acide (méth)acrylique non réagi obtenu à l'étape d), la fraction distillée riche en alcool obtenue à l'étape e).

La présente invention permet de surmonter les inconvénients de l'état de la technique. Elle fournit plus particulièrement un moyen de limiter les problèmes liés à la présence de sous-produits non valorisables dans un procédé de synthèse de (méth)acrylate de méthyle ou d'éthyle, les produits valorisables étant les réactifs non réagis ou l'ester recherché (produits nobles), ou les sous-produits lourds tels que les oligomères d'acide acrylique qui sont capables de libérer des produits nobles.

Ceci est accompli par le choix de conditions opératoires, en particulier en effectuant la réaction d'estérification avec un rapport molaire acide/alcool compris entre 1,05 et 4, de préférence entre 1,05 et 3, et sous une pression allant de la pression atmosphérique à 5 bars.

En outre, en minimisant la quantité d'eau entrant dans le réacteur d'estérification, la réduction de la quantité de sous-produits lourds et en particulier d'adduits formés au cours de la synthèse est encore accentuée.

Les inventeurs ont en effet découvert, que dans ces conditions, les sous-produits non valorisables sont générés en quantité moindre, ce qui induit les avantages suivants pour le procédé global : une valorisation beaucoup plus simple des produits lourds ou ayant un point d'ébullition plus élevé que l'acide acrylique (en particulier les oligomères d'acide acrylique), une simplification du train de purification finale de l'ester formé incluant un nombre restreint de colonnes de distillation, et une consommation énergétique moindre car la quantité d'alcool à recycler est plus faible.

Ainsi, l'invention permet de mettre en oeuvre une installation simplifiée pour produire les (méth)acrylates de méthyle ou d'éthyle avec une haute pureté et optimise le bilan matières du procédé.

### BREVE DESCRIPTION DES FIGURES

- La Figure 1 représente de manière schématique une installation pour la mise en oeuvre du procédé selon l'invention, appliqué en particulier à la synthèse de l'acrylate d'éthyle.
- La Figure 2 illustre le gain énergétique pour le recyclage de l'éthanol obtenu avec le procédé selon l'invention.

### EXPOSE DETAILLE DE L'INVENTION

L'invention est maintenant décrite plus en détail et de façon non limitative dans la description qui suit.

Selon l'invention, le rapport molaire acide/alcool fait référence aux teneurs en acide et en alcool de l'ensemble des flux alimentant le réacteur d'estérification (flux de produits purs et flux recyclés).

Selon un mode de réalisation préféré, le rapport molaire acide (méth) acrylique/alcool dans le réacteur est compris entre 1,05 et 3, plus préférentiellement entre 1,5 et 3, voire entre 2 et 2,5.

Selon un mode de réalisation préféré, la pression est choisie entre la pression atmosphérique et 3 bars.

La réaction d'estérification est réalisée en présence d'un catalyseur acide, par exemple une résine échangeuse de cations acides dans le cas d'une catalyse hétérogène ; ou à titre de catalyseur dans le cas d'une catalyse homogène, on peut utiliser par exemple de l'acide sulfurique, ou un acide organique sulfonique, tel que l'acide méthane sulfonique, l'acide para-toluène sulfonique, l'acide benzène sulfonique, l'acide dodécyl sulfonique, ou leurs mélanges.

La réaction est effectuée en présence d'un ou plusieurs inhibiteurs de polymérisation qui sont introduits dans le réacteur, à raison de 500 à 5000 ppm par rapport au mélange brut réactionnel. Comme inhibiteurs de polymérisation utilisables, on peut citer par exemple la phénothiazine, l'hydroquinone, l'éther monométhylique d'hydroquinone, le diterbutyl para-crésol (BHT), la paraphénylène diamine, le TEMPO (2,2,6,6-tétraméthyl-1-piperidinyloxy), le di-tertiobutylcatéchol, ou les dérivés du TEMPO, tel que le OH-TEMPO, seuls ou leurs mélanges en toutes proportions. Un ajout supplémentaire d'inhibiteur de polymérisation est généralement effectué au niveau du traitement ultérieur de purification.

En général, de l'eau est introduite dans le réacteur en quantité non négligeable sous différentes formes :
D'une part, la qualité d'alcool industriel utilisé comme réactif comprend en général de l'eau, à une teneur de l'ordre de 5 à 10% massique.

D'autre part, les flux contenant de l'alcool et/ou de l'acide non réagis provenant du train de purification du milieu réactionnel comprenant le produit recherché avec de l'eau, sont en général recyclés à la réaction. Le flux de recyclage de l'alcool peut alors comprendre de 10 à 60% massique d'eau, le flux de recyclage de l'acide peut comprendre de 2 à 20% d'eau.

Selon un mode de réalisation préféré de l'invention, on peut limiter la quantité d'eau à l'entrée du réacteur, en mettant en place un système de déshydratation sur un ou plusieurs de ces flux.

Le système de déshydratation peut être tout système apte à séparer l'eau d'un milieu plus ou moins complexe, connu de l'homme de l'art.

On peut citer à titre d'exemple une unité de séparation membranaire, plus particulièrement une unité de déshydratation par pervaporation ou par perméation de vapeur. Selon ce mode de réalisation, l'unité de déshydratation par séparation membranaire peut comporter une membrane inorganique, de préférence zéolite, et de manière plus particulièrement préférée zéolite de type T ; ou une membrane polymérique, de préférence une membrane hydrophile à base d'alcool polyvinylique.

Alternativement, on peut utiliser un système par adsorption sous pression modifiée (dénommé aussi PSA pressure swing adsorption) tel que celui connu pour la déshydratation des alcools.

Ou encore, il est possible d'éliminer l'essentiel de l'eau par simple distillation.

Il est entendu qu'il est possible d'utiliser différents systèmes de déshydratation dans un même procédé.

Selon ce mode de réalisation, il est possible de réduire de l'ordre de 85% l'eau introduite dans le réacteur, ce qui abaisse la teneur en eau en entrée du réacteur à environ 0,8-1,5% massique au lieu de 5-10% massique en l'absence d'unité de déshydratation. Cette réduction est significative pour avoir un impact bénéfique sur la formation d'adduits au cours de la synthèse.

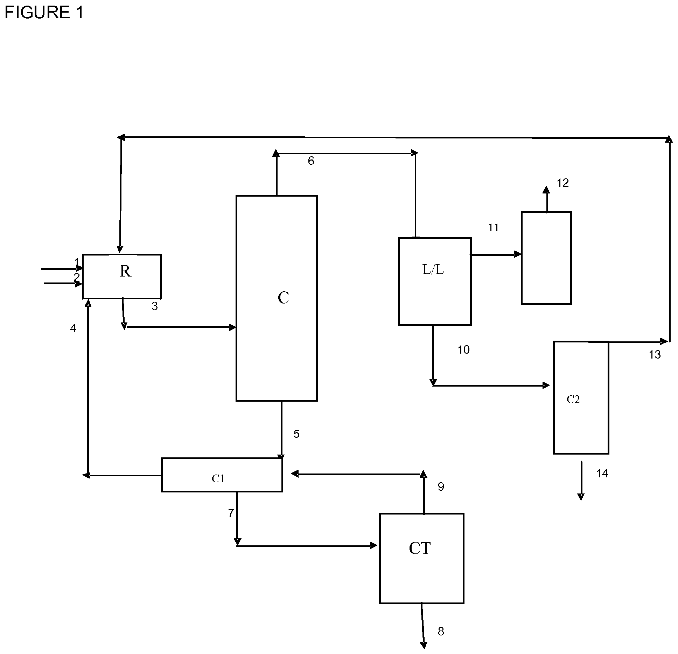

En faisant référence à la Figure 1, une installation de production d'acrylate d'éthyle selon l'invention comporte un réacteur R. Le réacteur R est alimenté par une conduite d'amenée d'acide acrylique 1, une conduite d'amenée d'éthanol 2. Le réacteur contient préférentiellement un catalyseur de type résine échangeuse de cations acides. Dans le cas d'une catalyse homogène, le réacteur est en outre alimenté par une conduite d'amenée de catalyseur (non représenté).

En sortie du réacteur, le mélange réactionnel 3 est envoyé vers une colonne à distiller C qui sépare, en pied, un flux 5 comportant essentiellement l'acide acrylique non réagi, des traces de produits légers (température d'ébullition inférieure à celle de l'acide acrylique), et les produits ayant une température d'ébullition plus élevée que l'acide acrylique (oligomères de l'acide acrylique et adduits de Michael), dénommés par la suite adduits, et, en tête, un flux 6 comportant l'acrylate d'éthyle formé et des produits plus légers que l'acide acrylique (éthanol non réagi, sous-produits tels que acétate d'éthyle, acide acétique).

Le flux 5 de pied de colonne C est envoyé vers une colonne à distiller C1 qui sépare un flux 4 comportant l'acide acrylique résiduel et les produits plus légers, ce flux 4 étant recyclé dans le réacteur R. Un flux 7 constitué essentiellement de produits lourds (adduits) est séparé de la colonne C1 et soumis à un craquage thermique dans le craqueur thermique CT.

Comme colonne à distiller C ou C1, on peut utiliser une colonne comprenant des internes du type garnissage vrac ou ordonné du type plateaux dual flow, perforés à déversoir ou à clapets. On peut aussi mettre en place au lieu de la colonne C1 un évaporateur à film.

Le craquage thermique permet de recycler les produits nobles (composés de départ ou produit fini) potentiellement récupérables de la fraction de produits lourds. Le craquage thermique est effectué à une température pouvant aller par exemple de 120°C à 220°C, éventuellement en présence d'un catalyseur acide tel que l'acide sulfurique ou un acide sulfonique . Le craquage thermique a pour effet de libérer les composés de départ, essentiellement l'acide acrylique, présents dans les adduits, mais a peu d'effet sur l'éthoxy propionate d'éthyle qui de ce fait reste présent en partie à la sortie du craqueur thermique dans le flux 9 d'acide acrylique qui est recyclé au niveau de la colonne C1, le reste de l'éthoxy propionate d'éthyle étant dans le flux 8 avec les autres produits lourds non valorisables, le flux 8 étant incinéré.

Le flux 6 de tête de la colonne à distiller C est envoyé vers une section d'extraction liquide / liquide (décanteur ou contacteur) pour générer, d'une part, une phase aqueuse 10 contenant de l'éthanol qui est recyclé à la réaction (flux 13) après distillation dans une colonne C2 (le flux aqueux 14 appauvri en éthanol pouvant être recyclé pour la phase d'extraction liquide/liquide), et d'autre part, une phase organique 11.

La section d'extraction liquide / liquide peut être constituée d'une colonne d'extraction liquide / liquide de type agitée ou colonne garnie, d'une batterie de mélangeur - décanteur, d'un ou plusieurs décanteurs en série.

La phase organique 11 peut être soumise à une ou plusieurs étapes complémentaires de distillation pour conduire à l'acrylate d'éthyle purifié 12. Cependant, le procédé selon l'invention permet de simplifier ce schéma final de rectification de l'ester recherché et ainsi il est possible d'atteindre plus facilement les spécifications souhaitées notamment en taux résiduel d'éthoxy propionate d'éthyle.

Les unités de déshydratation (non représentées sur la figure) visant à réduire la quantité d'eau introduite dans le réacteur, telles que décrites précédemment, peuvent être mises en place sur la ligne 2 d'amenée d'alcool frais, sur la ligne 12 de recyclage d'alcool, ou sur la ligne 4 de recyclage d'acide.

Les exemples suivants illustrent la présente invention et n'ont pas pour but de limiter la portée de l'invention telle que définie par les revendications annexées.

### PARTIE EXPERIMENTALE

### Exemple 1

Des essais de synthèse d'acrylate d'éthyle ont été réalisés un utilisant un réacteur ayant un diamètre de 2,5 cm, une hauteur de 55 cm et un volume de 270 ml. Le réacteur est rempli avec 330 ml de résine K1431 (LANXESS). Avant mise dans le réacteur, la résine (330 ml) a été conditionnée dans le mélange réactionnel entraînant une diminution du volume par remplacement de l'eau dans sa structure par les réactifs présents dans le mélange réactionnel.
Les réactifs sont introduits en tête du réacteur via une pompe. Ils peuvent être préchauffés à la température de réaction. En pied du réacteur se trouve un condenseur qui refroidit le mélange réactionnel grâce à une calandre alimentée en eau. La régulation de pression est faite par une action sur une vanne de régulation placée en sortie du condenseur.
Le mélange réactionnel est stabilisé avec 1000 ppm d'hydroquinone et mis sous agitation. Pour tenir compte des flux issus de recyclages, le réacteur a été alimenté par un mélange synthétique comprenant en % massiques :
1,5% d'eau, 65%-75% d'acide acrylique (AA), 20%-28%d'éthanol (EtOH), 2,5% à 4% d'acrylate d'éthyle (AE) et des impuretés tels que acétate d'éthyle (45 ppm), acide acétique et hydroquinone à hauteur de 1000 ppm.
La composition du mélange synthétique est réajustée de façon à faire varier le ratio molaire AA/EtOH de 1,5 à 2,5.
Le débit d'alimentation varie de telle façon que le temps de séjour (débit d'alimentation / volume de résine avant conditionnement) varie entre 45 min et 60 min. Le chauffage du réacteur et le préchauffage du mélange d'alimentation est fait pour que la réaction s'effectue à 75°C ou à 85°C, et la pression est fixée à 2 bars.

Des analyses sont effectuées par CPG et HPLC à partir de prélèvements de milieu réactionnel. La teneur en eau est déterminée par Karl Fisher et la teneur en acide acrylique peut être éventuellement déterminée par potentiométrie. Les résultats sont exprimés de la façon suivante :
- Conversion du réactif (AA ou EtOH), % = 100 - (nombre de moles de réactif restantes / nombre de moles de réactif introduites).
- Sélectivité en AE, % = 100 * nombre de moles d'AE produites / nombre de moles de réactif ayant réagies.
- Quantité en kg de sous-produits lourds formés (adduits) par tonne d'AE produite
- Quantité en kg d'adduits valorisables par tonne d'AE produite
- Quantité en kg d'adduits non valorisables par tonne d'AE produite
- Quantité en kg d'époxy propionate d'éthyle (EPE) formé par tonne d'AE produite

Les résultats des différents essais sont rassemblés dans les tableaux 1 à 3 ci-après.

**Tableau 1**

| Ratio molaire AA/ EtOH | Température (°C) | Temps de séjour (min) | Conversion EtOH (%) | Sélectivité AE par rapport à EtOH (%) |
|---|---|---|---|---|
| 1,5 | 85 | 60 | 72,8 | 97,1 |
| | | 45 | 69,6 | 97,3 |
| | 75 | 60 | 63,8 | 97,6 |
| | | 45 | 58,5 | 97,8 |
| 2 | 85 | 60 | 81,9 | 97,1 |
| | | 45 | 79,6 | 97,3 |
| | 75 | 60 | 73,8 | 97,8 |
| | | 45 | 68,7 | 97,9 |
| 2,5 | 85 | 60 | 86,6 | 97,3 |
| | | 45 | 84,5 | 97,5 |
| | 75 | 60 | 81 | 97,8 |
| | | 45 | 76,4 | 97,9 |

La conversion de l'éthanol varie de 58,5% à 86,6% et la sélectivité en acrylate d'éthyle par rapport à l'éthanol est toujours supérieure à 97%.

**Tableau 2**

| Ratio molaire AA/EtOH | Température (°C) | Temps de séjour (min) | Conversion AA (%) | Sélectivité AE par rapport à AA (%) |
|---|---|---|---|---|
| 1,5 | 85 | 60 | 47,2 | 97,7 |
| | | 45 | 45,5 | 98,1 |
| | 75 | 60 | 41,8 | 98,3 |
| | | 45 | 38,4 | 98,5 |
| 2 | 85 | 60 | 40,4 | 97 |
| | | 45 | 39,4 | 97,6 |
| | 75 | 60 | 37,1 | 98,1 |
| | | 45 | 34,2 | 98,3 |
| 2,5 | 85 | 60 | 36,6 | 96,5 |
| | | 45 | 34,9 | 97,3 |
| | 75 | 60 | 32,5 | 97,8 |
| | | 45 | 30,7 | 98,1 |

La conversion de l'acide acrylique varie de 30,7% à 47,2% et elle est liée à la présence en excès stoechiométrique du réactif acide. La sélectivité en acrylate d'éthyle par rapport à l'acide acrylique varie de 96,5% à 98,5%.

**Tableau 3**

| Ratio molaire | Température (°C) | Temps de séjour | Total Adduits | Adduits Valorisables | Adduits non valorisables | EPE |
|---|---|---|---|---|---|---|
| AA/EtOH | | (min) | (kg/t) | (kg/t) | (kg/t) | (kg/t) |
| 1,5 | 85 | 60 | 28,9 | 3,9 | 25 | 12 |
| | | 45 | 25,9 | 2,5 | 23,4 | 12 |
| | 75 | 60 | 22,3 | 2,6 | 19,7 | 10,5 |
| | | 45 | 20,2 | 2 | 18,2 | 10,1 |
| 2 | 85 | 60 | 33,8 | 9,6 | 24,2 | 10,1 |
| | | 45 | 29,4 | 6,8 | 22,5 | 10,1 |
| | 75 | 60 | 23,4 | 4,5 | 18,9 | 9,1 |
| | | 45 | 21,3 | 3,8 | 17,5 | 8,8 |
| 2,5 | 85 | 60 | 38,7 | 15,3 | 23,3 | 8,6 |
| | | 45 | 31,7 | 10,2 | 21,5 | 8,7 |
| | 75 | 60 | 25,4 | 7,3 | 18,1 | 7,9 |
| | | 45 | 22,9 | 5,9 | 17 | 7,9 |

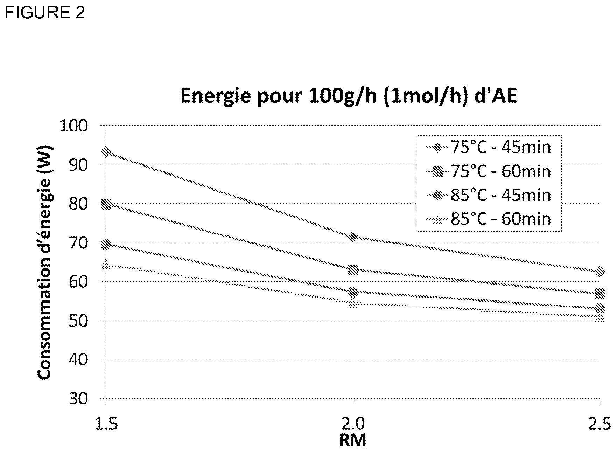

Le ratio molaire AA/EtOH influe sur la quantité totale des adduits formés lors de la réaction d'estérification. Lorsque l'acide acrylique est en excès dans le flux réactionnel, la teneur totale en adduits formés augmente. Cependant, il a été constaté qu'en même temps la quantité d'adduits non valorisables, en particulier l'éthoxy propionate d'éthyle, diminue lorsque le ratio molaire AA/EtOH augmente. La perte en matières premières liée à l'élimination des adduits non valorisables se trouve donc réduite lorsque l'on réalise l'estérification avec un large excès stoechiométrique d'acide acrylique.
En effet, en moyenne de 0,5 kg à 1 kg d'adduits non valorisables sont produits en moins par tonne d'acrylate d'éthyle produite, pour une augmentation du ratio molaire AA/EtOH de 0,5 ; Cette diminution, ramenée à une échelle industrielle par exemple à un atelier pouvant produire 100000 T/an d'acrylate d'éthyle, conduit à un gain de 50 T à 100 T de matières premières résultant directement de cette augmentation du ratio molaire.
La réduction de la formation d'éthoxy propionate d'éthyle va aussi produire des effets sur les étapes de purification finale de l'acrylate d'éthyle en facilitant l'obtention de la spécification recherchée pour la teneur en EPE résiduelle (généralement < 50 ppm).
Par ailleurs, la figure 2 illustre le gain énergétique obtenu par le procédé selon l'invention.

Le recyclage de l'éthanol non réagi nécessite de l'extraire du milieu réactionnel par de l'eau et de distiller la solution aqueuse d'éthanol. Dans le procédé selon l'invention, basé sur un excès de réactif acide, la quantité d'alcool non réagi se trouve réduite et en conséquence son recyclage peut être effectué avec une quantité d'énergie plus faible.
La figure 2 représente la consommation énergétique liée au recyclage de l'éthanol, ramenée à une production de 100 g/h d'acrylate d'éthyle, en fonction du ratio molaire (RM) AA/EtOH, dans les conditions de synthèse (température, temps de séjour) précédemment décrites. Le gain énergétique est toujours supérieur à 20% lorsque le RM varie de 1,5 à 2,5,

### Exemple 2 : Effet du ratio molaire Acide/alcool sur la formation d'éthoxy propionate d'éthyle

Pour illustrer l'effet bénéfique de l'excès d'acide acrylique sur la formation d'EPE, des essais comparatifs ont été réalisés dans le même appareillage que celui de l'exemple 1, avec un volume de résine K 1431 de 206 ml, une température réactionnelle de 75°C sous une pression de 1,3 bar et un temps de séjour de 110 min.
Le tableau 4 ci-après montre l'effet bénéfique du ratio molaire AA/EtOH sur la diminution de la formation d'EPE. On observe également l'effet défavorable du temps de séjour supérieur à une heure pour la formation d'EPE, (comparativement aux résultats du tableau 3 obtenus avec des temps de séjour inférieurs).

**Tableau 4**

| Ratio molaire | Température | Temps de séjour | EPE |
|---|---|---|---|
| AA/EtOH | (°C) | (min) | (kg/t) |
| 2,3 | 75 | 110 | 19 |
| 1,4 | 75 | 110 | 20 |
| 1 (comp) | 75 | 110 | 22,6 |
| 0,8 (comp) | 75 | 110 | 24 |
| 0,5 (comp) | 75 | 110 | 28 |
| 0,19 (comp) | 75 | 110 | 35 |

### Exemple 3 : Effet de l'eau sur la productivité du procédé

Des essais de synthèse d'acrylate d'éthyle ont été réalisés dans les mêmes conditions que celles de l'exemple 1, avec un flux d'alimentation composé, en % massiques, de 0,5 à 6% d'eau, 65%-75% d'acide acrylique, 20-28% d'éthanol, 0 à 3% d'acrylate d'éthyle et des impuretés tels que acétate d'éthyle (45 ppm), acide acétique et hydroquinone à hauteur de 1000 ppm.
On a fait varier la teneur en eau de façon à simuler un flux au moins partiellement déshydraté pour l'alimentation du réacteur.
Dans ces essais, la température de réaction est 75°C et le temps de séjour dans le réacteur est 45 min.
Les résultats des essais sont rassemblés dans le tableau 5 ci-après.

**Tableau 5**

| Ratio molaire AA/EtOH | H₂O % | AE % | Total Adduits (g/100g AE) | Adduits valorisables (%) | Conversion ETOH (%) | Sélectivité AE par rapport à EtOH (%) | Sélectivité AE par rapport à AA (%) |
|---|---|---|---|---|---|---|---|
| 2,5 | 0,6 | 0 | 2,2 | 26 | 76 | | |
| 2,5 | 3 | 0 | 2,5 | 26 | 69 | 97 | 97,5 |
| 2,5 | 5,7 | 0 | 2,7 | 23 | 61 | | |
| 2 | 1 | 3 | 2,1 | 17 | 69 | 97,9 | 98,3 |

Dans ces conditions, lorsque la teneur en eau dans le flux d'alimentation du réacteur d'estérification est réduite (de 5,7% qui représentent une teneur classique dans les procédés d'estérification, à 0,6% pouvant être obtenu après séparation membranaire de l'eau présente dans un flux recyclé), la conversion de l'alcool augmente (de 61% à 76%), la sélectivité en AE par rapport à l'éthanol ou l'acide acrylique est améliorée.
Il a été constaté que la quantité d'adduits formés diminue (2,7 à 2,2 g/100g d'AE produits) alors que la proportion d'adduits valorisables par exemple par traitement thermique augmente (23% à 26%). En conséquence, la formation d'éthoxy propionate d'éthyle non valorisable se trouve réduite, ce qui facilite le train de purification de l'acrylate d'éthyle et améliore le bilan matière du procédé.

## Revendications

1. Procédé de synthèse de (méth)acrylate de méthyle ou d'éthyle par estérification de l'acide (méth)acrylique avec l'alcool correspondant, en présence d'un catalyseur acide, **caractérisé en ce que** la réaction d'estérification est effectuée dans un réacteur avec un rapport molaire acide/alcool compris entre 1,05 et 4 sous une pression allant de la pression atmosphérique à 5 bars, et avec un temps de séjour du flux soumis à la réaction comprise entre 30 minutes et 1 heure.

2. Procédé selon la revendication 1 **caractérisé en ce que** le rapport molaire acide/alcool est compris entre 1,5 et 3.

3. Procédé selon la revendication 1 ou 2 **caractérisé en ce que** la pression va de la pression atmosphérique à 3 bars.

4. Procédé selon l'une quelconque des revendications précédentes **caractérisé en ce que** l'acide est l'acide acrylique.

5. Procédé selon l'une quelconque des revendications précédentes **caractérisé en ce que** l'alcool est l'éthanol.

6. Procédé selon l'une quelconque des revendications précédentes **caractérisé en ce que** la température de réaction est comprise entre 60°C et 90°C.

7. Procédé selon l'une quelconque des revendications précédentes **caractérisé en ce que** le réacteur est un réacteur à lit fixe ou un réacteur agité.

8. Procédé selon l'une quelconque des revendications précédentes **caractérisé en ce qu'**il comprend au moins une étape de déshydratation d'un flux alimentant le réacteur.

9. Procédé selon la revendication 8 **caractérisé en ce que** l'étape de déshydratation est appliquée à l'un au moins des flux suivants : le flux d'alimentation en alcool frais, un flux de recyclage de l'alcool non réagi, ou un flux de recyclage de l'acide non réagi.

10. Procédé selon la revendication 8 ou 9 **caractérisé en ce que** l'étape de déshydratation est effectuée par séparation membranaire, par distillation, ou par adsorption modulée en pression.

11. Procédé de synthèse de (méth)acrylate de méthyle ou d'éthyle par estérification de l'acide (méth)acrylique avec l'alcool correspondant, en présence d'un catalyseur acide **caractérisé en ce qu'**il comprend les étapes suivantes :
a) l'alimentation d'un réacteur en acide (méth)acrylique, en catalyseur acide et en alcool et la réaction d'estérification dans le réacteur telle que définie dans l'une quelconque des revendications précédentes ;
b) le soutirage d'un flux de (méth)acrylate de méthyle ou d'éthyle en sortie du réacteur ;
c) la distillation du flux de (méth)acrylate de méthyle ou d'éthyle, permettant de séparer, en tête, un flux de (méth)acrylate appauvri en sous-produits lourds et en acide (méth)acrylique non réagi, et en pied un flux comportant les sous-produits lourds, l'acide (méth)acrylique non réagi et des traces d'alcool et de produits légers ;
d) la séparation du flux de pied obtenu à l'étape c), en un flux comportant l'acide (méth)acrylique non réagi et des traces d'alcool et de produits légers, ce flux étant recyclé dans le réacteur, et en un flux de sous-produits lourds qui est soumis à un craquage thermique libérant un flux de produits valorisables qui retourne à l'étape d) ;
e) l'extraction liquide / liquide du flux de tête obtenu à l'étape c) par un flux aqueux permettant de séparer une phase organique purifiée de (méth)acrylate de méthyle ou d'éthyle, et une phase aqueuse, la phase aqueuse étant distillée pour récupérer d'une part une fraction riche en alcool qui est recyclée vers le réacteur, et d'autre part une fraction riche en eau qui est utilisée en tant que flux aqueux dans l'étape d'extraction liquide / liquide ;
f) éventuellement la déshydratation de l'un au moins des flux suivants : l'alimentation en alcool de l'étape a), le flux comportant l'acide (méth)acrylique non réagi obtenu à l'étape d), la fraction distillée riche en alcool obtenue à l'étape e).

12. Procédé selon la revendication 11 comprenant en outre une ou plusieurs étapes de distillation de la phase organique purifiée de (méth)acrylate de méthyle ou d'éthyle afin d'éliminer des composés organiques supplémentaires.

13. Procédé selon la revendication 11 ou 12 **caractérisé en ce que** l'étape de déshydratation est effectuée par séparation membranaire, par distillation, ou par adsorption modulée en pression.

## Patentansprüche

1. Verfahren zur Synthese von Methyl- oder Ethyl-(meth)acrylat durch Veresterung von (Meth)acrylsäure mit dem entsprechenden Alkohol in Gegenwart eines sauren Katalysators, **dadurch gekennzeichnet, dass** die Veresterungsreaktion in einem Reaktor mit einem Säure/Alkohol-Molverhältnis zwischen 1,05 und 4 unter einem Druck im Bereich von Normaldruck bis 5 bar und mit einer Verweilzeit des der Reaktion unterworfenen Stroms zwischen 30 Minuten und 1 Stunde durchgeführt wird.

2. Verfahren nach Anspruch 1, **dadurch gekennzeichnet, dass** das Säure/Alkohol-Molverhältnis zwischen 1,5 und 3 liegt.

3. Verfahren nach Anspruch 1 oder 2, **dadurch gekennzeichnet, dass** der Druck im Bereich von Normaldruck bis 3 bar liegt.

4. Verfahren nach einem der vorhergehenden Ansprüche, **dadurch gekennzeichnet, dass** es sich bei der Säure um Acrylsäure handelt.

5. Verfahren nach einem der vorhergehenden Ansprüche, **dadurch gekennzeichnet, dass** es sich bei dem Alkohol um Ethanol handelt.

6. Verfahren nach einem der vorhergehenden Ansprüche, **dadurch gekennzeichnet, dass** die Reaktionstemperatur zwischen 60 °C und 90 °C liegt.

7. Verfahren nach einem der vorhergehenden Ansprüche, **dadurch gekennzeichnet, dass** es sich bei dem Reaktor um einen Festbettreaktor oder einen Rührreaktor handelt.

8. Verfahren nach einem der vorhergehenden Ansprüche, **dadurch gekennzeichnet, dass** es mindestens einen Schritt der Dehydratisierung eines den Reaktor speisenden Stroms umfasst.

9. Verfahren nach Anspruch 8, **dadurch gekennzeichnet, dass** der Dehydratisierungsschritt auch auf mindestens einem der folgenden Ströme angewendet wird: den Einspeisungsstrom von frischem Alkohol, einen Rückführungsstrom von nicht umgesetztem Alkohol oder einen Rückführungsstrom von nicht umgesetzter Säure.

10. Verfahren nach Anspruch 8 oder 9, **dadurch gekennzeichnet, dass** der Dehydratisierungsschritt durch Membrantrennung, durch Destillation oder durch Druckwechseladsorption durchgeführt wird.

11. Verfahren zur Synthese von Methyl- oder Ethyl-(meth)acrylat durch Veresterung von (Meth)acrylsäure mit dem entsprechenden Alkohol in Gegenwart eines sauren Katalysators, **dadurch gekennzeichnet, dass** es die folgenden Schritte umfasst:
a) Einspeisung von (Meth)acrylsäure, saurem Katalysator und Alkohol in einen Reaktor und Veresterungsreaktion in dem Reaktor gemäß einem der vorhergehenden Ansprüche;
b) Abziehen eines Stroms von Methyl- oder Ethyl-(meth)acrylat am Ausgang des Reaktors;
c) Destillation des Stroms von Methyl- oder Ethyl-(meth)acrylat, was es ermöglicht, am Kopf einen Strom von (Meth)acrylat, der an schweren Nebenprodukten und nicht umgesetzter (Meth)-acrylsäure abgereichert ist, und am Sumpf einen Strom, der die schweren Nebenprodukte, nicht umgesetzte (Meth)acrylsäure und Spuren von Alkohol und leichten Produkten umfasst, zu trennen;
d) Trennung des in Schritt c) erhaltenen Sumpfstroms in einen Strom, der nicht umgesetzte (Meth)acrylsäure und Spuren von Alkohol und leichten Produkten umfasst, wobei dieser Strom in den Reaktor zurückgeführt wird, und in einen Strom von schweren Nebenprodukten, der einem thermischen Cracken unterworfen wird, bei dem ein Strom von Wertprodukten freigesetzt wird, der in Schritt d) zurückgeführt wird;
e) Flüssig/Flüssig-Extraktion des in Schritt c) erhaltenen Kopfstroms mit einem wässrigen Strom, was es ermöglicht, eine gereinigte organische Phase von Methyl- oder Ethyl(meth)-acrylat und eine wässrige Phase zu trennen, wobei die wässrige Phase destilliert wird, um einerseits eine alkoholreiche Fraktion, die zum Reaktor zurückgeführt wird, und andererseits eine wasserreiche Fraktion, die als wässriger Strom in dem Flüssig/Flüssig-Extraktionsschritt verwendet wird, zurückzugewinnen;
f) gegebenenfalls Dehydratisierung mindestens eines der folgenden Ströme: der Alkoholeinspeisung von Schritt a), des in Schritt d) erhaltenen Stroms, der nicht umgesetzte (Meth)-acrylsäure umfasst, oder der in Schritt e) erhaltenen alkoholreichen destillierten Fraktion.

12. Verfahren nach Anspruch 11, ferner umfassend einen oder mehrere Schritte der Destillation der gereinigten organischen Phase von Methyl- oder Ethyl(meth)acrylat zur Eliminierung von zusätzlichen organischen Verbindungen.

13. Verfahren nach Anspruch 11 oder 12, **dadurch gekennzeichnet, dass** der Dehydratisierungsschritt durch Membrantrennung, durch Destillation oder durch Druckwechseladsorption durchgeführt wird.

## Claims

1. A process for synthesizing methyl (meth)acrylate or ethyl (meth)acrylate by esterification of (meth)acrylic acid with the corresponding alcohol in the presence of an acidic catalyst, **characterized in that** the esterification reaction is carried out in a reactor at an acid/alcohol mole ratio of between 1.05 and 4, at a pressure ranging from atmospheric pressure to 5 bar, and with a residence time of the stream undergoing the reaction of between 30 minutes and 1 hour.

2. The process as claimed in claim 1, **characterized in that** the acid/alcohol mole ratio is between 1.5 and 3.

3. The process as claimed in claim 1 or 2, **characterized in that** the pressure ranges from atmospheric pressure to 3 bar.

4. The process as claimed in any one of the preceding claims, **characterized in that** the acid is acrylic acid.

5. The process as claimed in any one of the preceding claims, **characterized in that** the alcohol is ethanol.

6. The process as claimed in any one of the preceding claims, **characterized in that** the reaction temperature is between 60°C and 90°C,

7. The process as claimed in any one of the preceding claims, **characterized in that** the reactor is a fixed bed reactor or a stirred reactor.

8. The process as claimed in any one of the preceding claims, **characterized in that** it comprises at least one step of dehydrating a stream feeding the reactor.

9. The process as claimed in claim 8, **characterized in that** the dehydration step is applied to at least one of the following streams: the fresh alcohol feed stream, a recycling stream for the unreacted alcohol, or a recycling stream for the unreacted acid.

10. The process as claimed in claim 8 or 9, **characterized in that** the dehydration step is carried out by membrane separation, by distillation or by pressure swing adsorption.

11. A process for synthesizing methyl (meth)acrylate or ethyl (meth)acrylate by esterification of (meth)acrylic acid with the corresponding alcohol in the presence of an acidic catalyst, **characterized in that** it comprises the following steps:
a) feeding a reactor with (meth)acrylic acid, acidic catalyst and alcohol, and the esterification reaction in the reactor as defined in any one of the preceding claims;
b) drawing off a stream of methyl (meth)acrylate or ethyl (meth)acrylate at the reactor outlet;
c) distilling the stream of methyl (meth)acrylate or ethyl (meth)acrylate, making it possible to separate, at the top, a stream of (meth)acrylate depleted of heavy by-products and of unreacted (meth)acrylic acid, and at the bottom, a stream comprising the heavy by-products, unreacted (meth)acrylic acid and traces of alcohol and of light products;
d) separating the bottom stream obtained in step c) into a stream comprising unreacted (meth)acrylic acid and traces of alcohol and of light products, this stream being recycled into the reactor, and a stream of heavy by-products which is subjected to a thermal cracking which releases a stream of products with recoverable value which returns to step d);
e) liquid-liquid extraction of the top stream obtained in step c) by an aqueous stream making it possible to separate a purified organic phase of methyl (meth)acrylate or ethyl (meth)acrylate and an aqueous phase, the aqueous phase being distilled to recover, on the one hand, an alcohol-rich fraction which is recycled to the reactor, and on the other hand, a water-rich fraction which is used as aqueous stream in the liquid-liquid extraction step;
f) optionally dehydrating at least one of the following streams: the alcohol feed from step a), the flow comprising unreacted (meth)acrylic acid obtained in step d) or the alcohol-rich distilled fraction obtained in step e).

12. The process as claimed in claim 11, also comprising one or more steps for distilling the purified organic phase of methyl (meth)acrylate or ethyl (meth)acrylate in order to eliminate additional organic compounds.

13. The process as claimed in claim 11 or 12, **characterized in that** the dehydration step is carried out by membrane separation, by distillation or by pressure swing adsorption.
